# EUROPÄISCHE PATENTSCHRIFT

(11) **EP 0 379 543 B1**
(45) Veröffentlichungstag und Bekanntmachung des Hinweises auf die Patenterteilung: **08.03.1995**
(21) Anmeldenummer: 89907083.3
(22) Anmeldetag: 29.06.1989
(51) Int. Cl.: A61B 3/10

(54) **VORRICHTUNG ZUR BEOBACHTUNG VON OBJEKTEN UND ZUR ERFASSUNG DER TOPOGRAPHIE**
DEVICE FOR OBSERVING OBJECTS AND FOR RECORDING THE TOPOGRAPHY
DISPOSITIF D'OBSERVATION D'OBJETS ET D'ENREGISTREMENT TOPOGRAPHIQUE

(30) Priorität: 29.06.1988 DE 3821974; 08.10.1988 DE 3834293; 08.10.1988 DE 3834314
(43) Veröffentlichungstag der Anmeldung: 01.08.1990
(73) Patentinhaber: G. RODENSTOCK INSTRUMENTE GMBH, 85521 Ottobrunn (DE)
(72) Erfinder: WILMS, Karl, Heinz, D-8089 Emmering (DE); KLINGBEIL, Ulrich, D-8000 München 81 (DE); PLESCH, Andreas, D-8000 München 40 (DE)
(74) Vertreter: Münich, Wilhelm, Dr.
(86) Internationale Anmeldenummer: DE8900427
(87) Internationale Veröffentlichungsnummer: WO9000024

(56) Entgegenhaltungen:
- WO-A-83/02717
- WO-A-88/03396
- DE-A- 3 041 178
- US-A- 4 402 601

## Beschreibung

### Technisches Gebiet

Die Erfindung bezieht sich auf eine Vorrichtung zur Erfassung der Topographie eines Objekts, bei der der Bildaufbau mittels eines "Scanning-Verfahrens" erfolgt, und insbesondere auf eine Vorrichtung zur Beobachtung des Augenhintergrunds gemäß dem Oberbegriff des Patentanspruchs 1.

### Stand der Technik

Beispielsweise bei der Beobachtung der hinteren Augenabschnitte besteht die Schwierigkeit, daß die Beleuchtung und die Beobachtung durch die Augenpupille und die häufig optisch nicht klaren vorderen Augenmedien erfolgen muß, an denen Reflexe auftreten, und die Abbildungsfehler erzeugen.

Es ist deshalb bereits seit längerem vorgeschlagen worden, zur Beoachtung der hinteren Augenabschnitte anstelle von konventionellen Funduskameras scannende bzw. abtastende Vorrichtungen zu verwenden, bei denen der Augenhintergrund nicht großflächig ausgeleuchtet wird, sondern die mit einem auf einem möglichst kleinen Fleck fokussierten Beleuchtungslichtstrahl den Augenhintergrund abtasten und das reflektierte Licht in Zuordnung zur Abtastsequenz erfassen. Hierzu wird beispielsweise auf "The Foundations of Ophtalmology, Band 7, S. 307/308, Jahrgang 1962, die US-PS 4 213 678, die japanische Patentveröffentlichung 50-138822 sowie die EP-A-0 145 563 verwiesen.

Diese bekannten Vorrichtungen zur Beobachtung von Objekten und insbesondere zur Beobachtung der hinteren Augenabschnitte haben zwar den Vorteil, daß aufgrund des "Scanning-Prinzips" ein von Abbildungsfehlern, die beispielsweise durch getrübte vordere Augenmedien hervorgerufen werden können, weitgehend freies Bild der hinteren Augenabschnitte erhalten wird, dieses Bild ist aber im Gegensatz zu dem von anderen bekannten Vorrichtungen, wie beispielsweise Spaltlampen-Mikroskopen, gelieferten Bild kein Stereo-Bild.

Darüberhinaus ist es nicht möglich, das beispielsweise auf einem Monitor dargestellte Bild quantitativ auszuwerten. Damit ist es mit den vorstehend genannten Vorrichtungen nicht möglich, beispielsweise die Topographie des Augenhintergrundes zu erfassen.

Aus der WO 88/03396 ist eine Weiterbildung der vorstehend beschriebenen abtastenden Vorrichtungen zur Beobachtung des Augenhintergrundes bekannt, bei der unter anderem die Detektoreinrichtung mehrere Detektoren aufweisen kann. Aber auch diese bekannte Vorrichtung, von der im übrigen bei der Formulierung des Oberbegriffs des Patentanspruchs 1 ausgegangen worden ist, erlaubt noch nicht die Aufnahme eines Stereo-Bildes des Augenhintergrundes.

### Beschreibung der Erfindung

Der Erfindung liegt die Aufgabe zugrunde, eine Vorrichtung zur Erfassung der Topographie eines Objekts gemäß dem Oberbegriff des Patentanspruchs 1 derart weiterzubilden, daß es möglich ist, mit einfachen Mitteln die Topographie des aufzunehmenden Objekts zu erfassen, und die insbesondere ein Stereobild und zusätzlich zum Bild des Augenhintergrundes quantitative Daten über die Struktur des Augenhintergrundes liefern kann.

Eine erfindungsgemäße Lösung dieser Aufgabe ist mit ihren Weiterbildungen in den Patentansprüchen gekennzeichnet.

Überraschenderweise gelingt eine Lösung der erfindungsgemäß gestellten Aufgabe dadurch, daß von einer Vorrichtung zur Beobachtung von Objekten gemäß dem Oberbegriff des Patentanspruchs 1, d. h. von einer mit einer Beleuchtungslichtquelle und einer Abtasteinrichtung arbeitenden Vorrichtung ausgegangen wird.

Diese Vorrichtung wird erfindungsgemäß dadurch weitergebildet, daß zur Erfassung der Topographie des Objekts zeitsequentiell oder gleichzeitig wenigstens zwei Bilder des Objekts mit gegeneinander verschobenen Pupillen des Beleuchtungs- und/oder Beobachlungsstranlengangs aufnehmbar sind, und daß die Auswerte- und Synchronisiereinheit die mit unterschiedlicher Pupillenlage aufgenommenen Bilder miteinander verknüpft.

Dabei ist es insbesondere möglich mehr als zwei sich überlappende Pupillen zu verwenden, und die mit unterschiedlicher Blickrichtung aufgenommenen Bilder mit einen redundanten Algorithmus zu verknüpfen, so daß man zuverlässigere Aussagen über die Topographie beispielsweise des Augenhintergrundes erhält.

Selbstverständlich ist es aber auch möglich, zur Erzeugung eines stereoskopischen Bildes nur zwei Bilder aufzunehmen und hierzu die optische Achse des Beobachtungs- und/oder des Beleuchtungslichtstrahls in einer zur Pupillenebene konjungierten Ebene zu verschieben. Die mit unterschiedlicher Lage der optischen Achse erzeugten Bilder Werden zur Erzeugung eines Stereobildes überlagert.

Dabei kann die Stereowirkung dadurch wesentlich erhöht werden, daß die zur Erzeugung eines Stereobildes erfindungsgemäß getroffenen Maßnahmen sowohl auf den Beleuchtungslichtstrahl als auch auf den Beobachtungslichtstrahl wirken.

Erfindungsgemäß kann diese Verschiebung der optischen Achse des Beobachtungs- und/oder Beleuchtungslichtstrahls in einer zur Pupillenebene konjungierten Ebene beispielsweise durch eine Parallelverschiebung der optischen Achse mittels eines Allen-Separators oder durch die Verkippung eines Elements des optischen Systems der erfindungsgemäßen Vorrichtung erfolgen.

Beispielsweise kann ein Austrittsfenster oder ein sonstiges zur Bildebene konjungiertes optisches Element verkippbar sein.

Ferner ist es möglich, daß zum Umschalten zwischen unterschiedlichen Lagen der Beobachlungspupillen vor den Detektor Blenden vorgesehen sind. Auch können zur Realisierung der Verschiebung der Nachweispupillen wenigstens zwei voneinander beabstandete Detektoren vorgesehen werden oder die Verschiebung der Beleuchtungspupille und der Beobachtungspupille gleichsinnig erfolgen.

Bei einer Weiterbildung sind im Nachweisstrahlengang wenigstens ein Linsenelement mit Zylinderwirkung und ein Quadrantendetektor vorgesehen, der in der Mittelebene der beiden Fokus-Ebenen des Zylinder-Linsenelements angeordnet ist.

Die Auswerte- und Synchronisiereinheit wertet die Intensitätsverteilung auf dem Quadrantendetektor aus. Die Vorrichtung arbeitet damit wie folgt:
Das im Beobachtungsstrahlengang vorgesehene astigmatische System liefert in der meridionalen bzw. sagittalen Bildebene zwei Strichfoki, die einen bestimmten axialen Abstand aufweisen. Da sich in der Mitte zwischen den beiden Bildebenen der Quadrantendetektor befindet liegen die beiden Strichfoki dann, wenn der abtastende Laserstrahl exakt auf die reflektierende Fläche fokussiert ist, in gleichem Abstand vor und hinter dem Quadrantendetektor. Damit ergibt sich in diesem Falle eine kreisförmige Intensitätsverteilung in der Detektorebene.

Befindet sich dagegen die abgetastete Fläche außerhalb der Fokusebene des abtastenden Laserstrahls, so ergibt sich je nach Größe der Abweichung eine ovale Intensitätsverteilung auf dem Quadrantendetektor, deren Orientierung von der Richtung der Abweichung, d.h. davon, ob die Fokusebene des abtastenden Laserstrahls vor oder hinter dem Augenhintergrund liegt, und deren Exzentrizität vom Betrag der Abweichung abhängt.

Diese Asymetrie kann durch geeignete arithmetische Verknüpfung der vier Quadranten des Quadrantendetektors nachgewiesen werden.

Damit liefert das Ausgangssignals des Quadrantendetektors eine Tiefeninformation, die entweder zur Kompensation der Fokusabweichung (Anspruch 14), d.h. zur Realisierung eines Autofokussystems, oder auch zur Darstellung einer Information über die Topographie des Augenhintergrunds verwendet werden kann.

Diese Information kann dabei entweder aus der zur Fokussierung erforderlichen Verschiebung des im Beleuchtungsstrahlengangs vorgesehenen verschiebbaren Elements, die beispielsweise ein an sich bekannter Wegaufnehmer erfaßt, oder aus der Exzentrizität des Signals auf dem Quadrantendetektor abgeleitet werden (Anspruch 15).

Gleichzeitig kann durch additive Verknüpfung der vier Sektoren des Quadrantendetektors die Gesamtreflektivität des abgetasteten Objekts ermittelt werden, also eine reguläres Reflexionsbild erhalten werden.

Die erfindungsgemäße Vorrichtung liefert damit zusätzlich zu einem Bild des Augenhintergrunds eine Information über die Topographie des Augenhintergrunds, d.h. eine Information über die 3-dimensionale Gestaltung des Augenhintergrunds.

Der Meßbereich und die Tiefenauflösung der erfindungsgemäßen Vorrichtung kann durch geeignete Wahl der Brennweiten der sphärischen Linsenelemente (Anspruch 16) und des astigmatischen Linsenelements im Nachweisstrahlengang an die jeweiligen Verhältnisse leicht angepaßt werden.

### Kurze Beschreibung der Zeichnung

Die Erfindung wird nachstehend anhand von Ausführungsbeispielen unter Bezugnahme auf die Zeichnung näher beschrieben, in der zeigen:
- Fig. 1: eine Aufsicht auf eine erfindungsgemäße Vorrichtung,
- Fig. 2a: einen Ausschnitt aus einer erfindungsgemäßen Vorrichtung mit einem Allen-Separator,
- Fig. 2b: die zugehörige Pupillenteilung,
- Fig. 3: die Blenden in der Pupillenebene bei Verwendung von voneinander beabstandeten Pupillen, und
- Fig. 4a: den Strahlengang bei einer simultanen Scanningstereoskopischen Bildaufnahme, und
- Fig. 4b: die zugehörige Pupillenteilung,
- Fig. 5a und 5b: Pupillenteilungen bei Verwendung von mehr als zwei Pupillen, und
- Fig. 6: eine weitere Ausgestaltung einer erfindungsgemäßen Vorrichtung.

### Beschreibung von Ausführungsbeispielen

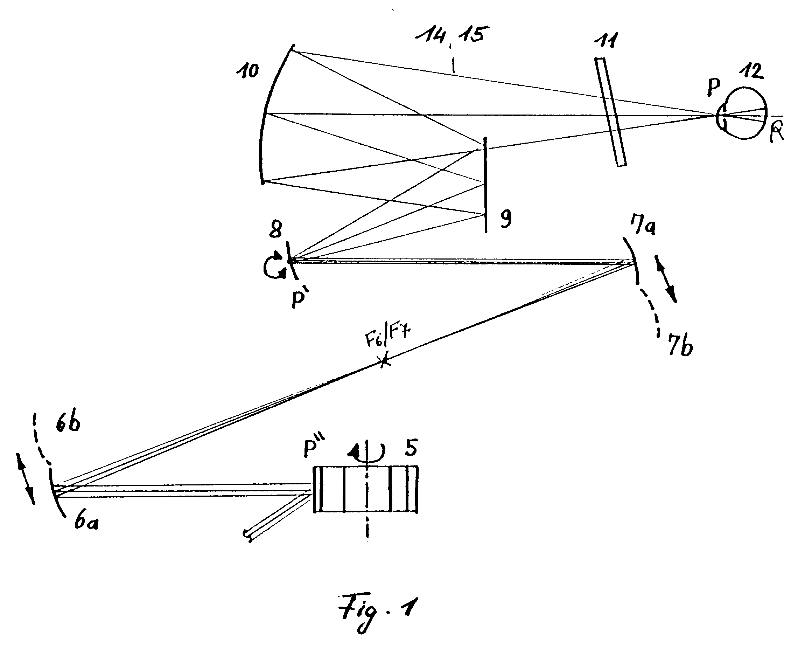

Die in Fig. 1 ausschnittsweise dargestellte erfindungsgemäße Vorrichtung weist eine nicht dargestellte Beleuchtungs-Lichtquelle, beispielsweise einen Laser, sowie eine nicht dargestellte Detektoreinrichtung auf, deren Ausgangssignal von einer Auswerte- und Synchronisiereinheit ausgewertet und beispielsweise auf einem Monitor dargestellt wird. Bei dem gezeigten Ausführungsbeispiel "verlaufen" sowohl der Beleuchtungslichtstrahl 14 als auch der vom Augenhintergrund kommende Lichtstrahl 15 über die Ablenkeinrichtung.

Der Lichtstrahl 14 des Lasers wird von einem ersten Ablenkelement (Horizontal-Scanner), der bei dem gezeigten Ausführungsbeispiel ein sich drehender Polygonspiegel 5 ist, in Horizontalrichtung (senkrecht zur Zeichenebene) abgelenkt. Der nun in der Horizontalebene aufgefächerte Strahl durchläuft das Spiegelsystem 6 und 7, und trifft auf ein zweites Ablenkelement (Vertikal-Scanner), das bei dem gezeigten Ausführungsbeispiel ein Schwing- bzw. Galvanometerspiegel 8 ist, auf. Nach dem Spiegel 8 hat das Strahlbündel einen "rechteckigen" Querschnitt. Nach Umlenkung an einem Planspiegel 9 wird er von einem Konkavspiegel 10 über ein weiter unten noch beschriebenes Element 11 auf das zu untersuchende Auge 12 abgebildet. Der reflektierte Lichtstrahl 15 durchläuft in umgekehrter Reihenfolge die genannten Elemente und wird nach dem Horizontal-Ablenkelement 5 von einem nicht dargestellten Detektor nach vorheriger Trennung des Beleuchtungs- und des Beobachtungslichtwegs nachgewiesen.

Erfindungsgemäß wird nun von folgendem Grundgedanken ausgegangen:
Bei "Scanning-Verfahren" wird die Beobachtungsrichtung und damit die Stereoparallaxe im wesentlichen durch die auf einen kleinen Teil der Pupille beschränkte Beleuchtung mit dem kollimierten Laserstrahl 14 festgelegt.

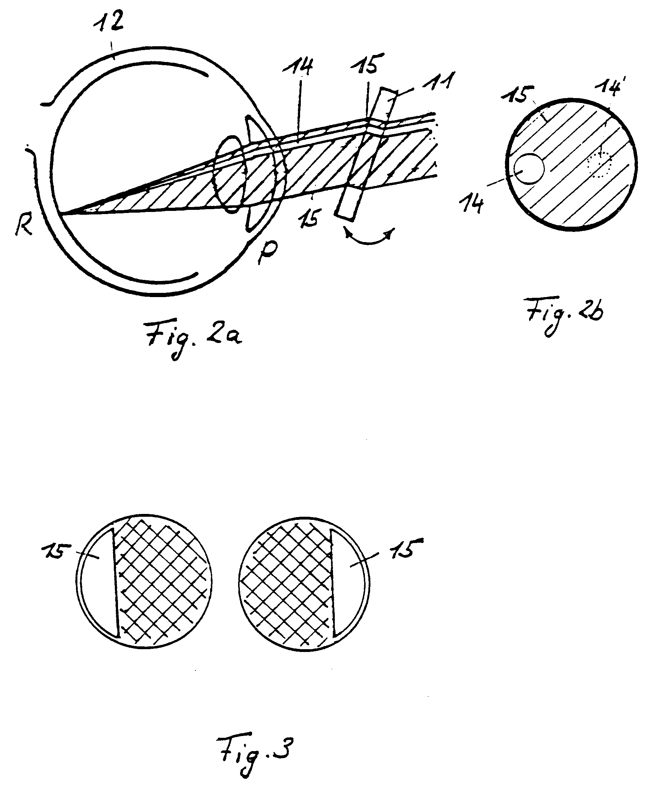

Fig. 2a zeigt, daß eine Parallelverschiebung des Laserstrahls 14 relativ zur Pupille P eine Veränderung der Orientierung des Lichteinfalls auf dem Augenhintergrund bewirkt und damit stereoskopische Bildunterschiede liefert. Fig. 2b zeigt die "Pupillenteilung" in der Pupillenebene P zwischen dem Beleuchtungslichtstrahl 14, dem Strahl 14' in der konjugierten Stereoposition und dem Beobachtungslichtstrahl 15.

Technisch kann eine derartige Pupillenteilung beispielsweise durch eine Parallelverschiebung der optischen Achse des Systems relativ zur Pupille P u.a. durch einen Allen-Separator, durch Verkippen des Austrittsfensters 11 oder durch Verkippen eines der Spiegel des optischen Systems realisiert werden.

Der verkippte Spiegel sollte möglichst konjugiert zur Bildebene R und möglichst wenig konjugiert zur Pupillenebene P sein, damit er die gewünschte optische Wirkung entfalten kann. Ein diese Bedingungen erfüllender Spiegel ist beispielsweise der Spiegel 9.

Bei einer erfindungsgemäßen Vorrichtung, bei der sowohl der Beleuchtungslichtstrahl 14 als auch das am Augenhintergrund reflektierte Licht 15 über die x/y-Scanningelemente geführt wird, kann eine Vergrößerung der Stereowirkung durch Kombination des Beleuchtungs-Stereoeffekts mit einem "Beobachtungs-Stereoeffekt" erreicht werden:

Der Beobachtungs-Stereoeffekt kann dabei mit den aus klassischen Ophthalmoskopen bekannten Mitteln erzielt werden. Die Kombination der "beiden Stereoeffekte" entspricht dabei einer Vergrößerung der Stereobasis über den Pupillendurchmesser.

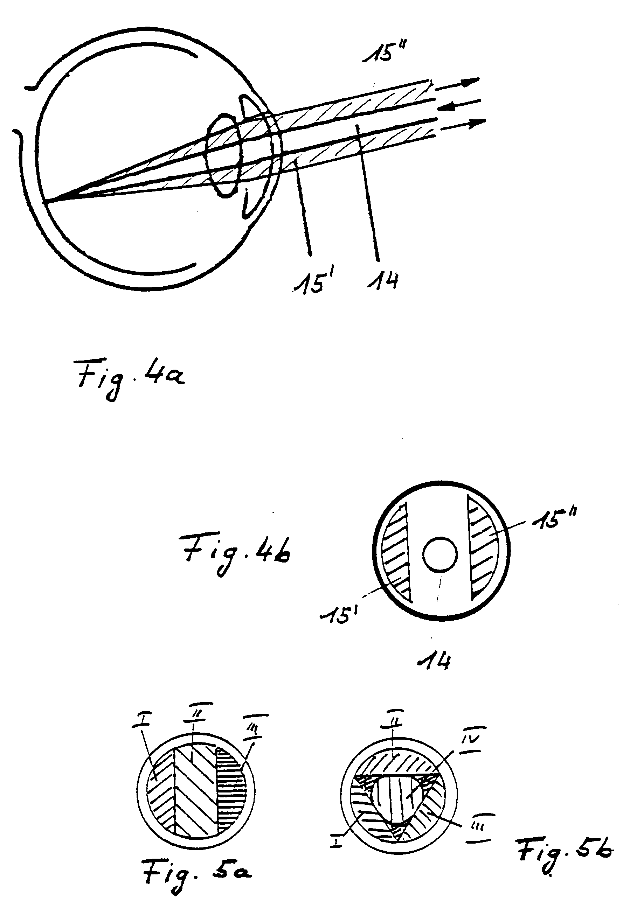

Hierzu werden die beiden Stereobilder nacheinander aufgenommen. Während der Aufnahme des ersten Bildes muß der Beleuchtungsstrahl auf der einen Pupillenseite liegen, während der Aufnahme des zweiten Stereobildes auf der anderen Pupillenseite (Fig. 3). Zwischen den Aufnahmen muß möglichst schnell mit den beispielsweise aus der Beschreibung zu Fig. 2 bekannten Mitteln umgeschaltet werden. Die zeitsequentiellen Stereobilder werden dann entsprechend elektronisch überlagert oder mit elektronischer Bildauswertung ausgewertet.

Gleichzeitig mit der Verschiebung der Lage des Beleuchtungslichtstrahles 14 in der Pupillenebene P wird - wie Fig. 3 zeigt - die Lage der Nachweispupillenebene gegensinnig verschoben. Dies kann beispielsweise durch einen austauschbaren Satz von Blenden vor dem Nachweisdetektor in einer zur Pupille P konjugierten Ebene oder durch einen Satz entsprechend angeordneter Detektoren, die wahlweise zum Liefern des Bildsignals zugeschaltet werden, erreicht werden.

Darüberhinaus ist es selbstverständlich auch möglich, im Nachweisstrahlengang in einer zur Pupillenebene konjugierten Ebene mit bekannten Mitteln eine Stereoseparation durchzuführen. Dies ermöglicht die simultane Aufnahme von Stereobildern.

Fig. 4a zeigt den zugehörigen Beleuchtungsstrahlengang 14 und die beiden Beobachtungsstrahlengänge 15' und 15''.

Fig. 4b zeigt die beispielsweise mit einer entsprechenden Blende realisierte zugehörige Pupillenteilung.

Zur simultanen "Stereo-Aufnahme" werden zwei simultan arbeitende Detektoren benötigt, die gegeneinander verschobene Teilbereiche der Pupille P beobachten sowie gegebenenfalls ein Stereoprisma mit Blende oder dgl., das die Pupillenteilung vornimmt. Die Stereobasis ist in diesem Falle aber durch den Pupillendurchmesser beschränkt, so daß die Stereoparallaxe und die Stereoseparation davon abhängen, wie weit die beiden Teilbereiche gegeneinander verschoben werden können.

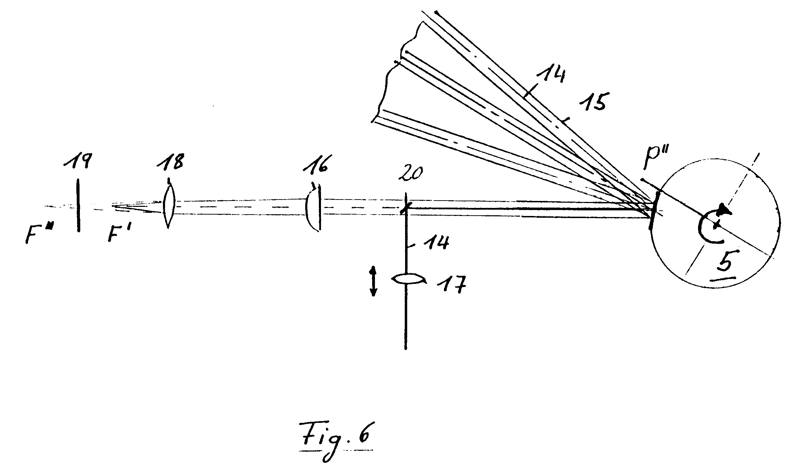

In den Fig. 5a und 5b ist die Anordnung von drei bzw. vier sich überlappenden Pupillen I-III bzw. I-IV dargestellt. Die Pupillen sind so ausgebildet, daß die Schwerpunkte der in den jeweiligen Strahlengängne definierten Pupillen gegeneinander verschoben sind.

Jeweils eine der Aufnahmen, deren Pupillenlage im Verhältnis zu den anderen eine Mittelstellung einnimmt, dient als Referenz bei der Auswertung. Es ergibt sich somit eine "Mehrbilder-Stereoskopie", die den Vorteil hat, daß die Ergebnisse zuverlässiger sowie die Auswertungsalgorithmen einfacher sind.

Darüberhinaus können die jeweiligen Halbbilder subjektiv bewertet oder mit Bildananlyse erfaßt und die Topographie mittels eines Rechners berechnet werden.

Fig. 6 zeigt ein weiteres Ausführungsbeispiel der Erfindung, von dem lediglich der vor dem Horizontal-Scanner 5 liegende Teil dargestellt ist. Der "hinter dem Scanner 5" befindliche Teil der Vorrichtung kann dabei entsprechend den Fig. 1 bis 5 oder auch in herkömlicher Weise ausgebildet sein.

Im Beleuchtungsstrahlengang 14 vor bzw. im Beobachtungsstrahlengang 15 nach dem Ablenkelement 5 ist ein Teilerspiegel 20 für die beiden Strahlengänge 14 und 15 angeordnet, der bei dem gezeigten Ausführungsbeispiel ohne Beschränkung des allgemeinen Erfindungsgedankens zu einer invertierten Gullstrand-Pupille führt. Im Nachweisstrahlengang 15 sind nach dem Teilerspiegel 20 ein Linsenelement 16 mit Zylinderwirkung, ein sphärisches Linsenelement 18 Wirkung und ein Quadrantendetektor 19 vorgesehen. Der Quadrantendetektor 19 ist in der Mittelebene der beiden Fokus-Ebenen F' und F'' des Zylinder-Linsenelements 16 angeordnet. Die nicht dargestellte Auswerte- und Synchronisiereinheit wertet die Intensitätsverteilung auf dem Quadrantendetektor aus. Ferner ist im Beleuchtungsstrahlengang 14 "vor" dem Teilerspiegel 20 ein in Pfeilrichtung verschiebbares Linsenelement 17 vorgesehen.

Die erfindungsgemäße Vorrichtung arbeitet damit wie folgt:
Das im Nachweisstrahlengang vorgesehene astigmatische System 16 liefert in der meridionalen bzw. sagittalen Bildebene F' bzw. F'' zwei Strichfoki, die einen bestimmten axialen Abstand aufweisen. Da sich in der Mitte zwischen den beiden Bildebenen der Quadrantendetektor 19 befindet, liegen die beiden Strichfoki dann, wenn der abtastende Laserstrahl exakt auf die reflektierende Fläche fokussiert ist, in gleichem Abstand vor und hinter dem Quadrantendetektor. Damit ergibt sich in diesem Falle eine kreisförmige Intensitätsverteilung in der Detektorebene.

Befindet sich dagegen die abgetastete Fläche außerhalb der Fokusebene des abtastenden Laserstrahls, so ergibt sich je nach Größe der Abweichung eine ovale Intensitätsverteilung auf dem Quadrantendetektor 19, deren Orientierung von der Richtung der Abweichung, d.h. davon, ob die Fokusebene des abtastenden Laserstrahls vor oder hinter dem Augenhintergrund liegt, und deren Exzentrizität vom Betrag der Abweichung abhängt.

Diese Asymetrie kann durch geeignete arithmetische Verknüpfung der vier Quadranten des Quadrantendetektors nachgewiesen werden.

Damit liefert das Ausgangssignals des Quadrantendetektors eine Tiefeninformation, die entweder zur Kompensation der Fokusabweichung, d.h. zur Realisierung eines Autofokussystems, oder auch zur Darstellung einer Information über die Topographie des Augenhintergrunds verwendet werden kann.

Diese Information kann dabei entweder aus der zur Fokussierung erforderlichen Verschiebung des im Beleuchtungsstrahlengangs vorgesehenen verschiebbaren Elements 17, die beispielsweise ein an sich bekannter Wegaufnehmer erfaßt, oder aus der Exzentrizität des Signals auf dem Quadrantendetektor abgeleitet werden.

Gleichzeitig kann durch additive Verknüpfung der vier Sektoren des Quadrantendetektors die Gesamtreflektivität des abgetasteten Objekts ermittelt werden, also ein reguläres Reflexionsbild erhalten werden.

Die erfindungsgemäße Vorrichtung liefert damit zusätzlich zu einem Bild des Augenhintergrunds eine Information über die Topographie des Augenhintergrunds, d.h. eine Information über die 3-dimensionale Gestaltung des Augenhintergrunds.

Darüberhinaus kann das Ausgangssignal zur Fokussierung und damit zur Verbesserung der Auflösung der erfindungsgemäßen Vorrichtung verwendet werden.

Der Meßbereich und die Tiefenauflösung der erfindungsgemäßen Vorrichtung kann durch geeignete Wahl der Brennweiten der sphärischen Linsenelemente 18 und des astigmatischen Linsenelements 16 im Nachweisstrahlengang an die jeweiligen Verhältnisse leicht angepaßt werden.

Darüberhinaus kann die erfindungsgemäße Vorrichtung auch zur Bestimmung der Cornea-Topographie verwendet werden.

Vorstehend ist die Erfindung anhand von Ausführungsbeispielen ohne Beschränkung des allgemeinen Erfindungsgedankens beschrieben worden. Insbesondere können die einzelnen beschriebenen Maßnahmen miteinander kombiniert werden.

## Patentansprüche

1. Vorrichtung zur Erfassung der Topographie eines Objektes mit
- einem Beleuchtungsstrahlengang (14), gebildet aus einer Beleuchtungslichtquelle, deren Licht auf einen zu beobachtenden Abschnitt (R) des Objekts (12) fokussiert ist, sowie einer Abtasteinrichtung (5,8), die eine Abtastbewegung des Lichtstrahls der Beleuchtungslichtquelle über den zu beobachtenden Abschnitt erzeugt und die strahlablenkende sowie strahlabbildende optische Elemente (6,7,9,10,17) aufweist,
- einem Beobachtungsstrahlengang (15), gebildet aus teilweise mit den optischen Elementen des Beleuchtungsstrahlenganges übereinstimmenden strahlablenkenden sowie strahlabbildenden optischen Elementen (6,7,9,10,16,18), die ein Bild des zu beobachtenden Objektabschnitts auf einer Detektoreinrichtung (19) erzeugen, und
- einer Auswerte- und Synchronisiereinheit, die aus dem zeitsequentiellen Ausgangssignal der Detektoreinrichtung ein Bild des zu beobachtenden Abschnitts erzeugt, dadurch **gekennzeichnet**, daß Mittel vorgesehen sind, die zur Erfassung der Topographie des Objekts zeitsequentiell oder gleichzeitig wenigstens zwei Bilder des Objekts mit gegeneinander verschobenen Pupillen des Beleuchtungs- und/oder Beobachtungsstrahlengangs aufnehmen, und
- daß die Auswerte- und Synchronisiereinheit derart ausgestaltet ist, daß sie die mit unterschiedlicher Pupillenlage aufgenommenen Bilder miteinander korrelliert.

2. Vorrichtung nach Anspruch 1
dadurch **gekennzeichnet**, daß Mittel (6,7,9,11) vorgesehen sind, die die optischen Achsen des Beleuchtungsstrahlengangs für die mit unterschiedlicher Pupillenlage aufgenommenen Bilder in der Pupillenebene gegeneinander verschieben.

3. Vorrichtung nach Anspruch 1 oder 2,
dadurch **gekennzeichnet**, daß Mittel vorgesehen sind, die zur Erzeugung eines stereoskopischen Bildes die optische Achse des Beobachtungs- und/oder Beleuchtungsstrahlenganges in einer Pupillenebene verschieben, und daß die mit unterschiedlicher Lage der optischen Achse(n) erzeugten Bilder zur Erzeugung eines Stereobildes überlagerbar sind.

4. Vorrichtung nach Anspruch 3,
dadurch **gekennzeichnet**, daß eine Parallelverschiebung der optischen Achse des Beleuchtungsstrahlenganges durch einen Allen-Seperator oder durch die Verkippung eines optischen Elements (11) im Beleuchtungsstrahlengang erfolgt.

5. Vorrichtung nach Anspruch 4,
dadurch **gekennzeichnet**, daß ein Austrittsfenster der Vorrichtung verkippbar ist.

6. Vorrichtung nach Anspruch 4,
dadurch **gekennzeichnet**, daß ein zur Bildebene des Beobachtungsstrahlenganges konjungiertes optisches Element (9) verkippbar ist.

7. Vorrichtung nach einem der Ansprüche 1 bis 6,
dadurch **gekennzeichnet**, daß zum Umschalten zwischen unterschiedlichen Lagen der Pupille des Beobachtungsstrahlenganges vor dem Detektor Blenden vorgesehen sind.

8. Vorrichtung nach einem der Ansprüche 1 bis 7,
dadurch **gekennzeichnet**, daß zur Realisierung der Verschiebung der Pupille des Beobachtungsstrahlenganges wenigstens zwei Detektoren vorgesehen sind.

9. Vorrichtung nach einem der Ansprüche 1 bis 8,
dadurch **gekennzeichnet**, daß die Verschiebung der Pupille des Beleuchtungsstrahlenganges und des Beobachtungsstrahlenganges gegensinnig erfolgt.

10. Vorrichtung nach einem der Ansprüche 1 oder 7 bis 9,
dadurch **gekennzeichnet**, daß Mittel vorhanden sind, die derart ausgestaltet sind, daß mehr als zwei Bilder mit definierten Pupillen aufgenommen werden.

11. Vorrichtung nach einem der Ansprüche 1 bis 10,
dadurch **gekennzeichnet**, daß zusätzlich der Beleuchtungsstrahl räumlich modulierbar ist.

12. Vorrichtung nach Anspruch 11,
dadurch **gekennzeichnet**, daß die Modulation mit Gittern erfolgt.

13. Vorrichtung nach einem der Ansprüche 1 bis 12,
dadurch **gekennzeichnet**, daß im Beobachtungsstrahlengang ein Linsenelement (16) mit Zylinderwirkung vorgesehen ist und die Detektoreinrichtung aus einem Quadrantendetektor (19) besteht, der in der Mittelebene der beiden Fokusebenen des Zylinder-Linsenelements angeordnet ist, und daß die Auswerte- und Synchronisiereinheit derart ausgestaltet ist, daß sie die Intensitätsverteilung auf dem Quadrantendetektor auswertet.

14. Vorrichtung nach Anspruch 13,
dadurch **gekennzeichnet**, daß wenigstens im Beleuchtungsstrahlengang mindestens ein verschiebbares optisches Element (17) vorgesehen ist, dessen Verschiebung längs der optischen Achse die Auswerte- und Synchronisiereinheit derart steuert, daß die Intensitätsverteilung auf dem Quadrantendetektor annähernd kreisförmig ist.

15. Vorrichtung nach Anspruch 13 oder 14, die zur Beobachtung des Augenhintergrundes ausgelegt ist,
dadurch **gekennzeichnet**, daß die Auswerte- und Synchronisiereinheit derart ausgestaltet ist, daß sie aus der Exzentrizität der Intensitätsverteilung des Quadrantendetektors (19) und/oder der Verschiebung des verschiebbaren optischen Elements (17) die Topographie des Augenhintergrundes bestimmt.

16. Vorrichtung nach einem der Ansprüche 13 bis 15,
dadurch **gekennzeichnet**, daß im Beobachtungsstrahlengang zusätzlich ein Linsenelement (18) mit sphärischer Wirkung angeordnet ist.

## Claims

1. Device for the detection of the topography of an object with
- an illumination ray path (14), formed from an illumination light source whose tight is focused on a section (R) of the object (12) to be observed and a scanning device (5, 8) which ge nerates a scanning movement of the light ray of the illumination light source over the section to be observed and which possesses ray-deflecting and ray-imaging optical elements (6, 7, 9, 10. 17),
- an observation ray path (15), formed from ray-deflecting and ray-imaging optical elements (6, 7, 9. 10, 16, 18) partially in common with the optical elements of the illumination ray path and which generate an image of the object section to be observed on a detector unit (1 9), and - an evaluation and synchronisation unit which generates an image of the section to be observed from the temporally sequential output signal of the detector unit,
**characterised** by the fact that means are provided which, to detect the topography of the object. take at least two images of the object temporaily sequentially or simultaneously with pupils of the illumination and/or observation ray paths which are shifted with relation to each other, and
- in that the evaluation and synchronisation unit is designed in such a way that it correlates images taken with different pupil positions with one another.

2. Device in accordance with Claim 1, **characterised** by the fact that means (6, 7, 9, 11) are provided which shift the optical axes of the illumination ray path for images taken with different pupil positions with respect to each other in the pupil plane.

3. Device in accordance with Claim 1 or 2, **characterised** by the fact that means are provided which shift the optical axis of the observation and/or illumination ray path in one pupil plane to generate a stereoscopic image, and that the images generated with the different positions of the optical axis/axes can be superimposed to generate a stereo image.

4. Device in accordance with Claim 3. **characterised** by the fact that a parallel shift of the optical axis of the illumination ray path is performed by an Allen separator or by tilting an optical element (11) in the illumination ray path.

5. Device in accordance with Claim 4, **characterised** by the fact that an exit window of the device can be tilted.

6. Device in accordance with Claim 4, **characterised** by the fact that an optical element (9) conjugate to the image plane of the observation ray path can be tilted.

7. Device in accordance with any of Claims 1 to 6, **characterised** by the fact that stops are provided in front of the detector for switch between the different positions of the pupil of the observation ray path.

8. Device in accordance with any of Claims 1 to 7, **characterised** by the fact that at least two detectors are provided to realise the shift in the pupil of the observation ray path.

9. Device in accordance with any of Claims 1 to 8, **characterised** by the fact that the shifts in the pupil of the illumination ray path and the observation ray path are made in opposite directions.

10. Device in accordance with any of claims 1 and 7, to 9. **characterised** by the fact that means are provided which are designed in such a way that more than two images can be taken with defined pupils.

11. Device in accordance with any of Claims 1 to 10, **characterised** by the fact that in addition the illumination ray can be spatially modulated.

12. Device in accordance with Claim 11, **characterized** by the fact that the modulation is performed with gratings.

13. Device in accordance with any of claims 1 to 12, **characterised** by the fact that a lens element (16) with cylinder power is provided in the observation ray path and the detector device consists of a quadrant detector (19) which is situated in the central plane of the two focal planes of the cylinder lens element, and that the evaluation and synchronisation unit is designed in such a way that it evaluates the intensity distribution on the quadrant detector.

14. Device in accordance with Claim 13, **characterised** by the fact that at least in the illumination ray path at least one movable optical element (17) is provided whose shift along the optical axis is controlled by the evaluation and synchronisation unit in such a way that the intensity distribution on the quadrant detector is approximately circular.

15. Device in accordance with Claim 13 or 14, which is designed to observe the fundus of the eye, **characterised** by the fact that the evaluation and synchronisation unit is designed in such a way that it determines the topography of the fundus of the eye from the eccentricity of the intensity distribution of the quadrant detector (19) and/or the shift of the movable optical element (17).

16. Device in accordance with any of Claims 13 to 15, **characterised** by the fact that in the observation ray path a lens element (18) with spherical power is additionally positioned.

## Revendications

1. Appareil pour relever la topographie d'un objet, comprenant
- un faisceau d'éclairage (14) formé par une source lumineuse d'éclairage dont la lumière est focalisée sur un segment (R) à observer de l'objet (12), ainsi que par un dispositif d'analyse (5, 8) qui produit un mouvement de balayage du rayon lumineux de la source lumineuse d'éclairage sur le segment à observer et contient les éléments optiques (6, 7, 9, 10, 17) de déviation et de représentation du rayon,
- un faisceau d'observation (15) formé par des éléments optiques (6, 7, 9, 10, 16, 18) de déviation et de représentation du rayon, qui correspondent partiellement aux éléments optiques du faisceau d'éclairage et produisent une image du segment d'objet à observer sur un dispositif détecteur (19) et
- une unité d'exploitation et de synchronisation générant une image du segment à observer à partir du signal de sortie, séquentiel dans le temps, du dispositif détecteur,
**caractérisé** en ce que des moyens sont prévus qui, pour relever la topographie de l'objet, enregistrent, séquentiellement dans le temps ou simultanément, au moins deux images de l'objet avec des pupilles mutuellement décalées du faisceau d'éclairage et/ou du faisceau d'observation, et que l'unité d'exploitation et de synchronisation est réalisée pour qu'elle opère la corrélation entre elles des images enregistrées avec des positions de pupilles différentes.

2. Appareil selon la revendication 1, caractérisé en ce que des moyens (6, 7, 9, 11) sont prévus qui décalent mutuellement, dans le plan pupillaire, les axes optiques du faisceau d'éclairage pour les images enregistrées avec des positions de pupilles différentes.

3. Appareil selon la revendication 1 ou 2, caractérisé en ce que des moyens sont prévus qui décalent l'axe optique du faisceau d'observation et/ou du faisceau d'éclairage dans un plan pupillaire en vue de la génération d'une image en relief ou stéréogramme, et que les images produites avec des positions différentes de l'axe ou des axes optique(s) sont superposables pour la génération d'un stéréogramme.

4. Appareil selon la revendication 3, caractérisé en ce que l'axe optique du faisceau d'éclairage est déplacé parallèllement à lui-même par un séparateur d'Allen ou par le basculement d'un élément optique (11) dans le faisceau d'éclairage.

5. Appareil selon la revendication 4, caractérisé en ce qu'une fenêtre de sortie de l'appareil est basculante.

6. Appareil selon la revendication 4, caractérisé en ce qu'un élément optique (9) conjugué au plan d'image du faisceau d'observation est basculant.

7. Appareil selon une des revendications 1 à 6, caractérisé en ce que des diaphragmes sont prévus devant le détecteur pour la commutation entre des positions différentes de la pupille du faisceau d'observation.

8. Appareil selon une des revendications 1 à 7, caractérisé en ce que deux détecteurs au moins sont prévus pour réaliser le déplacement de la pupille du faisceau d'observation.

9. Appareil selon une des revendications 1 à 8, caractérisé en ce que les déplacements des pupilles du faisceau d'éclairage et du faisceau d'observation s'effectuent dans des sens contraires.

10. Appareil selon une des revendications 1 ou 7 à 9, caractérisé en ce que des moyens sont prévus pour l'enregistrement de plus de deux images par des pupilles définies.

11. Appareil selon une des revendications 1 à 10, caractérisé en ce que le rayon d'éclairage est en outre modulable dans l'espace.

12. Appareil selon la revendication 11, caractérisé en ce que la modulation s'effectue au moyen de réseaux.

13. Appareil selon une des revendications 1 à 12, caractérisé en ce qu'un élément-lentille (16) à effet de cylindre est prévu dans le faisceau d'observation et le dispositif détecteur est formé par un détecteur à quadrants (19) placé dans le plan médian entre les deux plans focaux de l'élément-lentille à effet de cylindre, et que l'unité d'exploitation et de synchronisation est réalisée pour l'exploitation de la distribution d'intensité sur le détecteur à quadrants.

14. Appareil selon la revendication 13, caractérisé en ce qu'au moins un élément optique mobile (17) est prévu dans le faisceau d'éclairage, élément dont le déplacement le long de l'axe optique commande l'unité d'exploitation et de synchronisation de manière que la distribution d'intensité sur le détecteur à quadrants soit approximativement circulaire.

15. Appareil selon la revendication 13 ou 14 conçu pour observer le fond d'oeil, caractérisé en ce que l'unité d'exploitation et de synchronisation est réalisée de manière qu'à partir de l'excentricité de la distribution d'intensité du détecteur à quadrants (19) et/ou du déplacement de l'élément optique mobile (17), elle détermine la topographie du fond d'oeil.

16. Appareil selon une des revendications 13 à 15, caractérisé en ce qu'un élément-lentille (18) à effet sphérique est placé en plus dans le faisceau d'observation.
